# EUROPEAN PATENT SPECIFICATION

(11) **EP 0 395 105 B1**
(45) Date of publication and mention of the grant of the patent: **10.03.1993**
(21) Application number: 90108111.7
(22) Date of filing: 27.04.1990
(51) Int. Cl.: C07C 309/81, C07C 303/22

(54) **Process for preparing perfluoroalkenyl sulfonyl fluorides**
Verfahren zur Herstellung von Perfluoroalkenyl-sulfonyl-Fluoriden
Procédé pour la préparation de fluorures de perfluoroalkenyl sulfonyle

(30) Priority: 28.04.1989 IT 2034089
(43) Date of publication of application: 31.10.1990
(73) Proprietor: AUSIMONT S.p.A., I-20121 Milano (IT)
(72) Inventor: Navarrini, Walter, Dr., I-20010 Boffalora Ticino (Milan) (IT); Modena, Silvana, Dr., I-20052 Monza (Milan) (IT)
(74) Representative: Barz, Peter, Dr.

(56) References cited:
- US-A- 3 041 317
- US-A- 3 714 245
- US-A- 3 718 627
- JOURNAL OF THE AMERICAN CHEMICAL SOCIETY. vol. 82, no. 23, 14 December 1960, GASTON, PA US pages 6181 - 6188; D.C. ENGLAND ET AL.: "REACTIONS OF FLUOROÖLEFINS WITH SULFUR TRIOXIDE."
- JOURNAL OF THE CHEMICAL SOCIETY. 1966, LETCHWORTH GB pages 1171 - 1179; R. E. BANKS ET AL.: "PERFLUOROALKYL DERIVATIVES OF SULPHUR. PART VIII. SYNTHESIS AND REACTIONS OF PERFLUOROETHYLENESULPHONYL FLUORIDE"

## Description

The present invention relates to a process for the preparation of perfluoroalkenyl sulfonyl fluorides, in particular of perfluorovinyl sulfonyl fluoride.

These perfluorinated compounds which contain the sulfonyl function are useful monomers for the preparation of high-molecular-weight polymers, which find several uses.

US-A-3,041,317 discloses a process for the synthesis of perfluoroalkenyl sulfonyl fluorides of formula

R_{f}-CF=CF-SO₂F

wherein R_{f} is F, a perfluoroalkyl radical or an omega-hydro-perfluoroalkyl radical. The starting materials for said synthesis are 2-hydro-perfluoroalkyl sulfonyl fluorides of general formula

R_{f}-CF₂-CFH-SO₂F

which are dehydrofluorinated in order to yield perfluoroalkenyl sulfonyl fluorides. The reaction is carried out in a plant operating under reduced pressure, inside which a stream of reactants flows towards a catalyst composed of chromium oxide supported on KCl, at temperatures of from 450 to 630°C.

In particular for vinyl-sulfonyl fluoride, the reaction is carried out at temperatures of from 508 to 517°C, resulting in a conversion of 51% and a yield of 61%, based on the converted reactants, per each individual run.

R.E. Banks in J. Chem. Soc. (C), 1966, reports a synthesis of perfluorovinyl sulfonyl fluoride, wherein a catalyst and operating conditions are used which are exactly the same as those disclosed in US-A-3,041,317, with the only difference that the catalytic bed is pre-heated at 510°C before the synthesis is carried out.

In the paper by R.E. Banks, the general reactivity of said molecule is furthermore described.

In both of the above processes, the starting products are 2-hydro-perfluoro-sulfonyl fluorides having the general formula

R_{f}-CHF-CF₂-SO₂F

which may be synthesized by means of the controlled hydrolysis of the corresponding sultones of formula 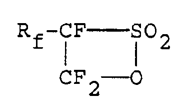
as described by D.C. England in J. Am. Chem. Soc. 1960, 82, 6181.

The main object of the present invention is the provision of a process for preparing perfluoroalkenyl sulfonyl fluorides, which can be carried out under temperature and pressure conditions which are more advantageous from an economic point of view than those of the processes known from the prior art.

Another object of the present invention is the provision of a simplified process which does not require additional steps, such as, e.g., the hydrolysis of sultone precursors, as in the processes of the prior art.

A further object is the provision of a process which uses readily available and inexpensive catalysts.

Finally, still a further object of the present invention is the provision of a process which affords high yields of the desired prfluoroalkenyl sulfonyl fluorides.

According to the present invention, these and still other objects which will become clear form the following disclosure may be achieved by a process for preparing perfluoroalkenyl sulfonyl fluorides having the general formula (I)

R_{f}-CF=CF-SO₂F (I)

wherein:
R_{f} is F or a perfluoroalkyl radical of form 1 to 9 carbon atoms,
which process comprises the contacting of a starting material comprising a perfluoroalkyl (sulfonyl fluoride) monofluoroacetyl fluoride of formula

R_{f}-CF₂-CF(COF)SO₂F (II)

wherein R_{f} is as defined above with a reactant selected from oxides of elements of the Groups IA, IIA, IIB, IIIA and IVA of the Periodic Table of Elements and mixtures thereof at a temperature of from about 150 to about 450°C, and recovering the desired compound of formula (I) from the reaction effluent.

The starting compound of formula (II) may be obtained by means of the isomerization of the corresponding sultone in the presence of catalytic amounts of KF or trialkylamines, with quantitative yields, according to the process described by D.C. England in J. Org. Chem. Soc. 1960, 82, 6181.

The reactant used in the instant process comprises one or more oxide(s) of the above mentioned elements, among which CaO, SiO₂ MgO, ZnO and mixtures thereof are generally preferred.

The reactions on which the instant process is based can be illustrated as follows for the case when CaO is used as the reactant:

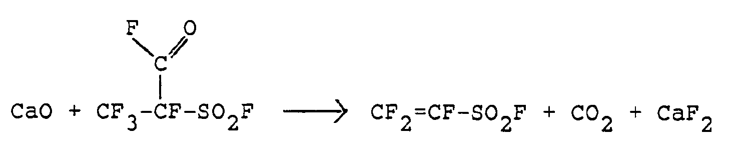

The amount of reactant used in the reaction generally is at least equal to the stoichiometric amount required for the above reaction. Preferably the reactant is employed in a substantial excess over said stoichiometric amount, which excess is also a function of the degree of comminution of the solid reactant used in the reaction.

The reactant advantageously is subjected to a preliminary activation by contacting it with the starting material before the actual process is carried out.

The process according to the present invention can advantageously be carried out continuously under atmospheric pressure, although pressures lower or higher than atmospheric pressure can be used as well.

The process temperature ranges from about 150 to about 450°C, preferably from about 180 to about 280°C.

The process is suitably carried out by causing a stream of an inert gas carrier saturated with vapours of the starting material used to flow over or through a bed of the reactant contained inside a glass or steel column reactor.

As the inert gas carrier, nitrogen is preferably used.

The process usually is carried out under anhydrous conditions, with both nitrogen and the reactant bed being previously dried thoroughly. The reactant bed may be dried by heating it at about 300-330°C for a few hours.

In the reactor, a packing of a particulate, e.g. siliceous, material with small particle size, is preferably used in addition to the reactant bed.

Flow rates of the carrier and starting material stream such as to secure a residence time of the starting material inside the reactor of from 3 to 30 seconds are generally used.

The effluent from the reactor may be condensed at a temperature of from 0 to -196°C in order to separate the reaction products from nitrogen, which may be discharged.

The condensate may be distilled under reduced pressure, e.g., under a pressure of about 10⁻³ torr, and the vapours may be condensed in a first condenser at a temperature of, e.g., from -100°C to -20°C in order to recover the desired perfluoroalkenyl sulfonyl fluoride, and then in a second condenser at -110°C in order to recover the produced SO₂.

The other byproducts, such as CO₂ and, possibly, SiF₄, as well as any decomposition products, such as CF₃COF or C₂F₄, may be removed by applying a dynamic vacuum during the distillation.

A complete conversion of the starting material may be obtained, with high yields, of the order of from 65 to 75%, which yields are defined as the ratio of the mols of the resulting desired product to the reacted mols of starting material.

The process according to the present invention is particularly useful for the preparation of compounds of formula (I) wherein R_{f} is F or a perfluoroalkyl radical of from 1 to 3 carbon atoms, particularly CF₃ and C₂F₅.

The following examples are to illustrate preferred embodiments of the present invention.

### EXAMPLE 1

In a continuously working plant, 3.15 g (13.5 mmol) of 2,3,3,3-tetrafluoro-2-(fluorosulfonyl)-propionyl fluoride of formula

CF₃-CF(COF)-SO₂F

were introduced into a loading "U"-shaped trap.

Said loading trap was then connected to the continuously working plant by means of valves.

A stream of previously thoroughly dried nitrogen was caused to flow (flow rate 10 litres/hour) through the loading trap which was kept at 0°C throughout the entire experiment.

Nitrogen saturated with the vapours of the starting material was passed through the reactor which was kept at 200°C.

Within about 2 hours, all of the starting material was passed through the reactor (a steel column having a diameter of 3.0 cm, packed with 100 g of calcium oxide and 100 g of small steel tubes).

The reactant bed thus prepared preliminarily was thoroughly dried by keeping it at 320°C for 4 hours, and was then activated by causing 13.5 mmol of the starting material to flow through it under the same operating conditions as described above.

The effluent gases from the reactor were passed through two traps maintained at -196°C in order to condense all of the reaction products, whereas the nitrogen carrier gas was discharged.

The crude reaction product contained in the two traps was distilled under a pressure of 10⁻³ torr. The vapours coming from the still kettle were caused to flow through cold traps kept at -80 and -110°C, respectively.

Inside the trap at -110°C, SO₂ condensed. In the trap at -80°C the desired product, trifluorovinyl sulfonyl fluoride of formula

CF₂=CF-SO₂F

was obtained. CO₂ and any decomposition products, such as, e.g., CF₃COF and C₂F₄ were removed by applying a dynamic vacuum during the distillation.
The conversion of the starting material was complete.

The yield, defined as the ratio of mols of the desired product (CF₂=CF-SO₂F) to the reacted mols of starting material, was 65%.

### EXAMPLE 2

By following the procedure of Example 1, 3.15 g (13.5 mmol) of 2,3,3,3-tetrafluoro-2-(fluorosulfonyl)-propionyl fluoride of formula

CF₃-CF(COF)-SO₂F

were caused to flow through the reactant bed, with the temperature of the loading trap being kept at 0°C throughout the entire experiment.

Within about 2 hours, all of the starting material was passed through the reactant bed of MgO, activated in the same way as disclosed in Example 1 and maintained at 200°C.

The flow rate of the carrier nitrogen stream was kept at 8 litres/ hour throughout the entire experiment.

The conversion of the starting material was 30%. The reaction yield, defined as in Example 1, was 63%.

## Claims

1. Process for preparing perfluoroalkenyl sulfonyl fluorides of general formula (I):
R_{f}-CF=CF-SO₂F (I)
wherein:
R_{f} is F or a perfluoroalkyl radical of from 1 to 9 carbon atoms,
which process comprises the contacting of a perfluoroalkyl (sulfonyl fluoride) monofluoroacetyl fluoride of formula (II):
R_{f}-CF₂-CF(COF)SO₂F (II)
wherein R_{f} is as defined above with a reactant selected from oxides of elements of the Groups IA, IIA, IIB, IIIA and IVA of the Periodic Table of Elements and mixtures thereof, at a temperature of from about 150 to about 450°C.

2. Process according to claim 1, wherein R_{f} is F or a perfluoroalkyl radical of from 1 to 3 carbon atoms.

3. Process according to any one of claims 1 and 2, carried out continuously and under atmospheric pressure.

4. Process according to any one of claims 1 to 3, wherein said reactant is selected from CaO, MgO, ZnO, SiO₂ and mixtures thereof.

5. Process according to any one of claims 1 to 4, wherein said temperature ranges from about 180 to about 280°C.

6. Process according to any one of claims 1 to 5, wherein said step of contacting with the reactant is carried out by causing a stream of an inert gas carrier saturated with the vapours of said compound of formula (II) to flow over or through the reactant.

7. Process according to claim 6, wherein said inert gas carrier is nitrogen.

## Patentansprüche

1. Verfahren zur Herstellung von Perfluoralkenylsulfonylfluoriden der allgemeinen Formel (I):
R_{f}-CF=CF-SO₂F (I)
worin
R_{f} für F oder einen Perfluoralkylrest mit 1 bis 9 Kohlenstoffatomen steht,
welches Verfahren umfaßt die Kontaktierung eines Perfluoralkyl(sulfonylfluorid)monofluoracetylfluorids der Formel (II):
R_{f}-CF₂-CF(COF)-SO₂F (II)
worin
R_{f} wie oben definiert ist, mit einem aus Oxiden von Elementen der Gruppen IA, IIA, IIB, IIIA und IVA des Periodensystems der Elemente und Mischungen davon ausgewählten Reaktanten, bei einer Temperatur von ungefähr 150 bis ungefähr 450°C.

2. Verfahren nach Anspruch 1, worin R_{f} für F oder einen Perfluoralkylrest mit 1 mit 3 Kohlenstoffatomen steht.

3. Verfahren nach irgendeinem der Ansprüche 1 und 2, kontinuierlich und unter atmosphärischem Druck durchgeführt.

4. Verfahren nach irgendeinem der Ansprüche 1 bis 3, worin der Reaktant aus CaO, MgO, ZnO, SiO₂ und Mischungen davon ausgewählt wird.

5. Verfahren nach irgendeinem der Ansprüche 1 bis 4, worin die Temperatur im Bereich von ungefähr 180 bis ungefähr 280°C liegt.

6. Verfahren nach irgendeinem der Ansprüche 1 bis 5, worin die Stufe des Kontaktierens mit dem Reaktanten ausgeführt wird, indem man einen Strom eines Inertgasträgers, der mit Dämpfen der Verbindung der Formel (II) gesättigt ist, veranlaßt, über oder durch den Reaktanten zu strömen.

7. Verfahren nach Anspruch 6, worin der Inertgasträger Stickstoff ist.

## Revendications

1. Procédé de préparation de fluorures de perfluoroalkényl-sulfonyle de formule générale (I):
R_{f}-CF=CF-SO₂F (I)
dans laquelle:
R_{f} est choisi dans le groupe comprenant F et les radicaux perfluoroalkyles comprenant 1 à 9 atomes de carbone,
comprenant le contact d'un fluorure monofluoroacétylique du fluorure de sulfonyle perfluoroalkylé de formule générale (II):
R_{f}-CF₂-CF(COF)-SO₂F (II)
dans laquelle:
R_{f} est tel que défini ci-dessus,
avec un réactif choisi parmi les oxydes des éléments appartenant aux groupes IA, IIA, IIB, IIIA et IVA de la Classification Périodique des Eléments ou de leurs mélanges, à une température comprise entre environ 150 et environ 450°C.

2. Procédé selon la revendication 1, dans lequel R_{f} est F ou un radical perfluoroalkyle contenant 1 à 3 atomes de carbone.

3. Procédé selon l'une quelconque des revendications 1 et 2, caractérisé en ce qu'il est effectué en continu et à pression atmosphérique.

4. Procédé selon l'une quelconque des revendications 1 à 3, caractérisé en ce que le réactif est choisi dans le groupe comprenant CaO, MgO, ZnO, SiO₂ et leurs mélanges.

5. Procédé selon l'une des revendications 1 à 4, dans lequel ledit domaine de température est compris entre 180°C environ et 280°C environ.

6. Procédé selon l'une quelconque des revendications 1 à 5, caractérisé en ce que l'étape de contact est réalisée par passage d'un courant gazeux comprenant un support inerte saturé par des vapeurs du composé de formule générale (II) sur ou au travers d'un lit dudit réactif.

7. Procédé selon la revendication 6, caractérisé en ce que le support inerte consiste en azote.
